# EUROPEAN PATENT APPLICATION

(11) **EP 2 428 157 A1**
(43) Date of publication of application: **14.03.2012**
(21) Application number: 10724985.6
(22) Date of filing: 07.01.2010
(51) Int. Cl.: A61B 1/07

(54) **INTUITIVE, MULTI-FUNCTION, ERGONOMIC ENDOSCOPIC SYSTEM WITH A SHEATH OF VARYING ELASTICITY AND A TIP WHICH FITS NATURAL ORIFICES**

(30) Priority: 06.11.2008 MX 2008014203
(71) Applicant: Campos Castellanos, Jorge Alberto, C.P. 52763 Huixquilucan, Estado de México (MX)
(72) Inventor: Campos Castellanos, Jorge Alberto, C.P. 52763 Huixquilucan, Estado de México (MX)
(74) Representative: Molina Garcia, Julia
(86) International application number: PCT/MX2010/000001
(87) International publication number: WO 2010/074549

(57) **Abstract**

An endoscopic system, apparatus and method for observing a dark area inside the body. The system includes an instrument of the endoscopic type, an image apparatus and a human apparatus interface. The endoscope includes a tip assembly which fits the natural orifices and is connected to a working sheath of varying elasticity which is joined to a intuitive and ergonomic gripping handle. The tip assembly includes a plurality of ports and an extension of the working channel for facilitating entry thereof, protecting the body tissues and protecting the optical image collector. The sheath assembly includes an active flexible segment which is connected to a passive flexible segment and the latter is in turn connected to a semi-rigid or rigid segment. An intuitive (instinctive perception) and ergonomic gripping handle is connected to the working sheath described, these operating moreover in an integral and coherent manner. The method for observing the dark area of the body cavity includes the steps and measures to be implemented in order to provide an instrument which has a segment of the working sheath with varying elasticity having an active flexible distal end and in order to gripping handle this segment at the desired angle of deflection.

## Description

### BACKGROUND OF THE INVENTION

Endoscopic instruments have been developed for over 100 years to explore, diagnose and treat obscured portions of cavities in the human body. These act as minimally invasive, usually through the natural orifices or on other occasions are introduced in the course of very small incisions in the skin. This produces little or nil disfigurement in human anatomy, minimal or no injury, with decreased costs and risks associated with conventional surgery using larger incisions and exposing the cavities for long periods of time. Endoscopic type instruments are constructed with a proximal portion that can be called the gripping gripping handle and a working sheath that varies in elasticity, being rigid, semirigid and flexible. These are introduced into the cavities of the body. In 1805 the first endoscope was developed and worked with candles for illumination it had different adapters at the front, which allowed for very a limited exploration. In 1876 the instruments acquire a shape similar to the ones at the present time and incorporate the Edison bulb, with a platinum filament. Thereafter there would be a deeper exploration into the cavities and initiate further search for the detail and expansion of diagnostic and therapeutic methods. Before 1980 in the urinary system, only the urethra, prostate and bladder could be explored. The single scan could then be made in the ureter was its catheterization and injection of contrast media for an x-ray or fluoroscopy observation.

From this decade, endoscopes were introduced thinner and longer in length that resulted in still a deeper visual exploration, this meant that the ureter (the tube that carries urine from the kidney towards the bladder and are two for each person with an average length of 24 to 26 cm) could be fully explored, although not in all cases due to the stiffness or hardness of the instrument working sheath since those were initially built with absolute rigidity due to the optics used and not allow any deflection as it could be damaged within the cavity under exploration and had relatively large diameters, resulting in several limitations to be introduced to explore the urinary system that is very smooth, has an internal diameter of approximately 3 mm and curves toward the kidney in the three planes . These instruments enabled to avoid open or conventional surgery and were referred to, as ureteroscopes and nephroscopes. This endoscopic armamentarium revolution began the era of minimally invasive surgery.

Subsequently, in the late 1980's, optical fibers were incorporated in rigid endoscopes. This allowed diameter reduction and permitted some deflection in the working sheath's instrument; therefore they were named semirrigid miniscopes and overcame some of the limitations of their previous generation. These rendered obsolete the rigid instruments that were introduced through the urethra into the kidney. Only rigid instruments for the exploration of the intrarrenal urinary system have prevailed and are called nephroscopes, which are inserted through the skin above the kidneys at the lower back, with a technique called percutaneous (through the skin). The latter still have the limitations of scanning various cavities intrarrenal and sometimes require two or more punctures to cross the kidney tissue and reach their cavities. At the same time of optical fibers integration in the semirigid miniscopes, flexible endoscopes emerged, which allowed endosurgical exploration and instrumentation of the intrarrenal urinary system, by being introduced from the urethra and avoiding in most cases, the percutaneous puncture.

Currently, rigid endoscopes are the ones most utilized in the lower urinary system, which is formed, by the urethra, prostate and bladder. Semirigid and flexible endoscopes are the most commonly used In the ureter and intrarrenal urinary system.

The advantages of flexible endoscopes are its adaptability, finesse and possibility to direct instrument's distal tip towards different cavities. Rigid and semirigid instruments do not allow this adaptability and flexibility inside the intrarrenal urinary system and prevent its exploration, so therefore endosurgically intervened. For example, using a rigid or even a semirigid instrument requires penetration through the lumbar region and even when they are introduced through natural channels, require excessive rotation and correction of natural curvatures. Rigid or semirigid endoscopes are made of hard materials; usually metallic that can injure or tear the urinary system especially during rotation maneuvers or by pushing forward. Therefore, examination within the natural anatomical curves of the urinary system ideally requires use of flexible instruments that can adapt and maneuver through, and to, the ureter passage instead of forcing the ureter passage to adapt to the shape of the instrument.

Flexible endoscopes have the above advantages, however its working sheath by being completely flexible makes insertion difficult and besides the already described anatomical features, there are natural areas smaller in diameter that should be overcome, requiring either to be dilated or greater rigidity from the instrument working sheath such as of a semirigid to be able to enter. Its construction is fragile because its fiber optics are unprotected from a rigid material such as metal, usually their construction is of a less consistent and firm material as they are typically manufactured out of soft plastic, polymer materials or alike wrapping the optical fibers, that are easily bendable. When trying to insert a flexible endoscope and find the natural obstructions mentioned above, it is necessary to push for progress. The lack of consistency renders easy damage to the instrument when fiber optics can be fractured, blocking out vision, as well as any other internal mechanism. Flexible instruments have another important point of fragility besides the distal zone for insertion in contact with the human body and are the connection of the gripping handle and the working sheath. Excessive flexibility of these instruments makes maneuverability difficult and most often it is necessary to use also the hands of another assistant to perform the insertion. Collaboration must occur synchronously and coordinated for the maneuver to be successful. For these reasons and the high cost of these instruments, most doctors prefer rigid and semirigid instruments. The skill required is usually above the typical average user experience so that most urologists do not have the skill, dexterity, feeling, or experience to handle fully flexible endoscopes.

In regard to the gripping handle attachment, rigid and semirigid endoscopes are not provided with an ergonomic gripping handle and should be gripping handled by the surgeon, through its optics, or the light and water conduits, in other words should be held by its components, which do not have a protective cover or housing. Flexible instruments are provided with a gripping handle, which is far from intuitive and poorly ergonomic. This means that the interrelation gripping handle-working sheath is not integral and coherent in regard to the work that should be performed, since at instrument's resting position, a lever is found at the proximal third, under the longitudinal axis, that activates the distal active flexible mechanism of the endoscope tip by lowering it by pushing forward or lifting it by retracting it. However the intrarrenal urinary system goes to the right and left during patients resting on their back and an instrument of this nature is to be utilized. These features prevent the instrument from being intuitive since mental processes should be carried out to define the various elements to behave a certain way, rather than when activated, respond so that is expected to be " natural ". In addition, these gripping handle grips produce encountered vectorial forces that alters, intuitiveness, ergonomicity and efficiency of the endoscope due to the disorderly location of their connectors which are the viewing optics-camera connection, light post with its cable, the input/output fluid port with its connecting tube. Another disadvantage of the instrument is that most of its weight is distributed towards the gripping handle, producing easy fatigue for the operator. It is also necessary to stand during the entire procedure for a better use of this instrument and it requires using a lead apron 12 to 15 kg throughout the operation time.

The disadvantage situations provided by current instruments have created the need to vision a surgical instrument that is friendly, lessens the difficulty of use, increases the probability of success, also intuitive, ergonomic, allowing the surgeon to be comfortable with his/her hands when handling it and being able to sit during the procedure should this be prolonged.

Therefore, the art has also sought a new generation of endoscopes that will improve and facilitate surgery upon the urinary system (including the upper urinary tract within the kidney, bladder, prostate and urethra) and minimize the risk of laceration and injury during surgical procedures. More specifically, the art has sought an instrument design that: can facilitate the insertion of the instrument into, and trough, a delicate non-linear cavity such as a urinary tract; facilitate the exploration of the upper urinary; facilitate exploration of the upper urinary tract in order to diagnose and surgically intervene anywhere in the urinary system; provides stability to avoid breakage during the procedure; provides easier mobility within the upper urinary system; and facilitates the introduction of different accessories with more precision.

The design and elements of a traditional face tip of an endoscopic type instrument, either rigid, semi-rigid or flexible, has changed very little since the first one was introduced. Basically they all include one or more of the following input/output ports: a working channel port to introduce operating accessories to perform a procedure; an optical image collector-conductor port, for example, a telescope port for viewing; a luminous conductor port, for example, an illumination fiber optics port; and sometimes an irrigation & suction channel port. The face tip is the first portion of the instrument to be in contact with the human body is crucial to introduce the instrument. The face tips of the instruments that exist already are hardly complementary to the shape of the orifices of the body to be explored, therefore its introduction leads to certain difficulties. In addition, since its inception the rigid and semirigid instruments used have points where the optic is ahead of the working channel. This means that accessories that emerge from the working channel of the instrument are out before they can have visual control, which causes a "blind spot". The impact is that when leaving a surgical accessory as laser fibers, tongs, baskets or scissors (endosurgical accessories) can cause inadvertent injury, tear or a hole in the adjacent tissue. In the case of the urinary system due to the intimate contact of the ureteric duct measuring 3 mm at its internal diameter and curvature features, this happens and the consequences can be dire. In those instruments referred, the endosurgical accessories emerge from its inferior portion and behind the optics, therefore, rotation of the instrument is necessary in order to properly accommodate and aim to perform with precision the required work. The twists and frequent rotations of the instrument increases the risk of perforation and / or inherent trauma, either during the insertion of the instrument or the pressure exerted by rectifying the natural curves, causing inflammation of the structures that are being explored. Flexible instruments have a flat face tip assembly with shapes that are far from being complementary to the openings through which instruments are inserted. In these the "blind spot" situation is less, however by having a different shape than the complementary entrance orifice, it produces difficulty in its insertion. Definitely the face tip assembly is of great importance for two things: 1. To facilitate the insertion of the instrument and 2. Protect the internal tissues of the accessories that emerge from the working channel. Therefore, the art has sought an endoscopic type instrument wherein the working tool or accessories exit at the face tip, coincident with or in front of the viewing device to reduce the risk of laceration by allowing the surgeon to view the instrumental accessories as they exit either in front of the optics, lenses, or from the midsection of the instrument face tip

Endoscopic type instruments have typically ranged in complexity from simple viewing scopes which employ a light source and an ocular system, to relatively complex instruments having a light source, an image collection system, fluid channels, and a surgical or working tool channel The required features employed in an endoscopic type instrument are determined in part by the requirements of the type of examination or surgery in which the instrument is used.

The light source for illuminating the site of interest is usually positioned outside the cavity. The light is communicated through the instrument by an illumination, or light conductor, usually formed of a fiber optic bundle. It is conceivable that the light conductor could be separate from the instrument itself. This would allow for use of an endoscope with a reduced diameter or would allow additional functions in a scope of a given diameter. No matter what additional use endoscopic type instruments have been put to, their examination properties remain their staple use. Conventional lenses for image collection and transmission generally require that the instrument be rigid or semi-rigid. Flexible endoscopic type instruments typically employ coherent optical fiber bundles wherein the opposite ends of the fibers are identically ordered. The image quality of lens based image collection and transmission is generally superior to image collection and transmission formed of fiber optics or fiber optics alone.

Endoscopic type instruments may be constructed to have fluid channels that may serve a variety of different purposes. For example, in certain procedures on the lungs, the fluid channel provides an air passage to allow the lung to breathe. In other procedures, the fluid channel may be used to insufflate, or inflate, a cavity in the body for better access to obtain a better view. In other procedures, a supply of cleansing fluid, such as water, may be used to clear away undesirable contaminant fluid, such as blood, from a location to facilitate inspection or to clean the image collector. A suction line is often used for removing fluids from the site. A working tool channel provides for the insertion of various working implements, or accessories, through the instrument such as forceps, scissors, punches, electrodes, laser and the like.

An endoscopic type instrument may include a typically tubular shaped working sheath connected to a gripping handle and viewing assembly, which typically provide a mechanical coupling to which a viewing apparatus is connected. The typical endoscopic type instrument may include fluid channels extending trough the working sheath, which communicate with external fluid connections on the gripping handle and the assembly. A working tool port on the gripping handle and viewing assembly typically communicates with a working tool channel in the working sheath and may include a clamp or other support device to hold the working tool in place. An illumination port typically communicates with a light source. The light is normally transmitted from the viewing end or proximal end of the instrument to a light directing lens, or lenses, at the distal end. An optical collector including an objective lens is positioned at the distal end and passes the image through the image conductor to the gripping handle and viewing apparatus through which the operator views the section of the cavity of interest. The objective lens, if used, is typically fixed and may be oriented along the longitudinal axis of the working sheath or be angled off-axis for a view to the side. Some endoscopic type instruments have a fixed combination of functions, while others may be adapted to allow a selection of functions from a variety of working tools and viewing methodologies.

The gripping handle and viewing apparatus of endoscopic type instrument usually accommodate various adapters for connecting various types of video, or other imaging, devices. In some cases, an image multiplexer is utilized to separate the image for simultaneous display on an optical viewer used for direct viewing and a video imager to televise or record the procedure.

An endoscopic type instrument having only a single optical collector-optical conductor or single telescope, alone, creates only a two-dimensional, or monoscopic, view of the region under inspection. This often results in a lack of depth perception for the user of the instrument, making it difficult to perform an accurate inspection or surgery. Three dimensional, or 3-D, viewing would allow for more precise viewing when maneuvering inside such anatomical features as the urinary tract, and would allow for better identification and perception of dimensions and distances from the instrument tip to the object in question, especially where the instrument is being used in a cavity containing a fluid. Although, three-dimensional, or stereoscopic, laparoscopic type instruments, such as stereomicroscopes, have been developed for creating a three-dimensional view of the object or region under inspection these are not suited for use in endoscopics. These instruments are provided with a pair of optical pathways of channels for transmitting a plurality of simultaneously gathered images of the object of interest to a stereoscopic viewer. Traditionally, the stereoscopic viewer has had microscope-like eyepieces through which the viewer views the respective images. The eyepieces are arranged so that the viewer's eyes provide the necessary convergence lo combine the images into a stereoscopic view. Convergence of right eye and left eye images of an object is done in normal stereopsis by converging the optical axes with the eyes or optical/mechanical means to accomplish convergence of the right and left images so that the brain receives and perceives the images as sufficiently close together for the brain to combine the images as a single three-dimensional image. The stereomicroscope is an example of such an optical/ mechanical device. Although the human brain can converge and "fuse" two separate views if the separation between the images is not too great, this is not easy or comfortable to achieve in practice. In typical stereo-microscopes, the problem is solved by using two converging optical systems. However, this is not a practical solution in endoscopic type systems where the necessary convergence at very short focal lengths is compounded by the need to keep the overall diameter of the system as small as possible so that the endoscope tube can be inserted through a single minimum size surgical incision, minimizing invasive procedures. Also, traditionally, where a video viewing system is used, the two parallel optical systems used in such arrangements do not converge the images and provide two separate images or video pictures.

Accordingly, prior to the development of the present invention, it is believed that there has been no endoscopic type instrument which: has the versatility of a flexible endoscope, while retaining the controllability of a semi-rigid or rigid endoscope; has an instrument working sheath which is both rigid for a portion of its length and flexible for a portion of its length; which avoids, or reduces, the necessity for rotation of the instrument when targeting is required and while working inside delicate cavities; provides three dimensional imaging; does not have a blind spot associated with the instrument when working tools or accessories exit the Instrument; also with an intuitive, ergonomic gripping handle which means that can be understood and its handling learned at first sight and use it from the first occasion; that with a rotor or gripping handler produces logical rotation of the distal active flexible tip towards the expected side such as if the rotor goes to the right, so is the active flexible tip and the same is for the left side; that at instrument's resting position it is directed towards the cavities of the renal system, left or right which is the natural position while lying on the back, when It is introduced in the urinary system for a diagnosis or treatment. Therefore, the art has sought an endoscopic instrument, or endoscope, which has the versatility of a flexible endoscope, while retaining the controllability of a semi-rigid or rigid endoscope; has an instrument working sheath which has a rigid portion and a flexible portion; prevents, or reduces, the necessity for rotation of the instrument when targeting is required and while working inside delicate cavities; provides three dimensional imaging in the viewing system; and does not have a blind spot at the point where the working tools or accessories exit the instrument; be of easy insertion, due to the face tip design which is complementary to the natural orifices; the gripping handle is intuitive and ergonomic by functioning and responding in a logical and expected manner while activating its mechanisms. The spirit or essence of the invention is to reveal a coherent gripping handle and working sheath instrument that makes it intuitive and with an ergonomic gripping handle. To achieve the goal of creating an intuitive instrument, the gripping handle and its relationship with the working sheath have to be related to the staple use of the cavity on which it will be utilized in such a manner that while activating the mechanisms or controls the instrument responds in a predictable manner, such as a natural act. Ergonomics, understood as the interrelation or comprehension of interactions of humans and other elements in a system, when applied to the present invention, is achieved in part by developing an instrument that at the gripping handle avoids encountered vectorial forces; it can be used during long periods of time, allows the user a comfortable position, Ex; sitting. It will allow the user a better and easier handling of the endoscopic type instrument that will render improved outcome in the patient.

The working sheath is provided with different elastic properties all along, thus multifunction, facilitating thee face tip assembly to be easily introduced, in such a manner that the active flexible segment can explore de non-linear cavities of the urinary system or any other one. The flexible passive segment fits into any explored system such as the urinary and aids the active flexible segment to easily reach any required area. The semi-rigid or rigid segment, and thus helps to propel forward the entrance of the above plus two segments, stabilize and facilitate their entry to prevent unwanted twisting of the proximal section of the working sheath. The above features help the instrument to be adapted inside the cavities with no or minimal rectification of body structures that are soft and non-linear.

### SUMMARY OF THE INVENTION

In accordance with the invention the foregoing advantages have been believed to be achieved through the endoscope, endoscope system, and method for viewing a portion of a body cavity of the present invention. The endoscope system of the present invention for viewing a visually obscured portion of a body cavity may include: an endoscope have a face tip assembly, having a plurality of input/output ports, associated with a working sheath assembly, the working sheath assembly being associated with a gripping handle and viewing assembly; the working sheath assembly including a working sheath having a distal end and an actively flexible working sheath segment disposed at the distal end of the working sheath for insertion into the cavity; a least one optical image collector adapted to gather an image from within the body cavity; at least one optical conductor, associated with the at least one optical image collector, and adapted to transmit the image to the gripping handle and viewing assembly; at least one luminous conductor adapted to provide illumination to the body cavity; at least one working channel disposed within the working sheath assembly adapted to permit a working instrument entry into the body cavity; and an imaging apparatus, associated with the at least one optical conductor, and adapted to capture the image to send it to a human interface apparatus adapted to permit viewing of the image.

Another feature of this aspect of the invention is that the endoscope system may include a working channel extension, associated with the face tip assembly, which includes al least one protrusion adapted to guide the working instrument and lo prevent impact of foreign matter located within the body cavity upon the at least one optical collector. An additional feature of this aspect of the present invention is that there may be two optical conductors for producing a three-dimensional image. A further feature of this aspect of the present invention is that the actively flexible working sheath segment may be disposed adjacent a passively flexible working sheath segment. An additional feature is that the passively flexible working sheath segment may be disposed adjacent a semi-rigid working sheath segment.

In accordance with the invention, the foregoing advantages have also been achieved through the present endoscope for viewing a portion of a body cavity. This aspect of the present invention may include: a face tip assembly, have a plurality of input/output ports, associated with a working sheath assembly, the working sheath assembly being associated with a gripping handle and viewing assembly; the working sheath assembly may include a longitudinal axis, a working sheath having a distal end and an actively flexible working sheath segment disposed at the distal end of the working sheath for insertion into the body cavity; al least one optical image collector adapted to gather an image from within the body cavity; at least one optical conductor, associated with the at least one optical image collector, and adapted to transmit the image to the gripping handle and viewing assembly; at least one luminous conductor adapted to provide illumination to the body cavity; and at least one working channel disposed within the working sheath assembly and adapted to permit a working instrument entry into the body cavity.

An additional feature of this aspect of the present invention is that the endoscope may include a working channel extension associated with the face tip assembly, which includes at least one protrusion to guide the working instrument and to prevent impact of foreign matter upon the at least one optical image collector. A further feature of this aspect of the present invention is that the working sheath assembly may have a longitudinal axis and the at least one optical image collector may lie in a first plane which is disposed substantially perpendicular lo the longitudinal axis of the working sheath assembly; and the al least one protrusion is disposed at the distal end of the working sheath forward of the first plane in which the at least one optical image collector lies, whereby the at least one optical image collector may view an operating tool passing forwardly beyond the al least one protrusion.

Another feature of this aspect of the present invention is that the plurality of input/output ports may include al least one operating tool port, and the operating tool port lies in a second plane which is disposed substantially parallel with the first plane in which the al least one optical collector lies. The first plane and the second plane may be substantially coplanar or the second plane be disposed in a spaced relationship from the first plane, toward the distal end of the working sheath.

A further aspect of the present invention is that the working sheath is connected to the gripping handle assembly, the gripping handle assembly presents with diverse features for the intuitive operation or functioning of the working sheath, particularly at its distal active end; the distal end that at instrument's resting position directs or deflects the active flexible segment, located at the distal end of the working sheath towards right or left, inside the body cavity which is the true position of the urinary system when under surgery. This motion is performed through a rotor or manipulator, which directs the active flexible segment at the distal end of the working sheath. This action of rotor motion towards right or left in synchrony with the active flexible segment of the working sheath is what confers the invention to be intuitive.

Another aspect of the present invention is that the gripping handle at its superior surface is provided by the input/output ports for irrigation/suction and of the working ports for working tool entrance; The gripping handle that has at its proximal end a channel for the at least one optical conductor of image or two image conductor when three dimension viewing is necessary; this output is placed at the proximal end and along the same longitudinal axis of the instrument. Another aspect of the present invention is that substantially under the channels and optical conductors and very close is found the exit for the luminous conductor connected or wired from inside the gripping handle to a cable or fiber optics for light conduction towards a light source for illumination. The latter to explain that the optical conductors and the luminous conductors exit in such a manner as being parallel and along the longitudinal axis of the endoscope type instrument of the present invention. These elements by being directed along the same path as the longitudinal axis of the instrument prevent vectorial forces against each other; thus allow an adequate behavior of the endoscope when rotation is required inside the body cavity.

Another features are that the gripping handle may have two optical image conductors for two image collectors that can render a three-dimensional viewing.

### BRIEF DESCRIPTION OF THE DRAWINGS

While some of the features, advantages, and benefits of the present invention, having been stated, others will become apparent as the description proceeds when taken in conjunction with the accompanying drawings in which:
FIG. 1 is a side view of an endoscope, such as a ureteroscope, cystoscope, in accordance with the present invention;
FIG.2 is a front view of a face tip assembly for use with the endoscope of FIG. 1;
FIG. 3 is a side view of the face tip assembly of FIG.2;
FIG. 4 is a perspective view of the face tip assembly of FIGS. 2 and 3;
FIG. 5 is a front view of another embodiment of a face tip assembly for use with the endoscope of FIG. 1;
FIG. 6 is a side view of the face tip assembly of FIG. 5;
FIG.7 is a front view of another embodiment of a face tip assembly for use with the endoscope of FIG. 1;
FIG.8 is a front view of another embodiment of a face tip assembly for use with the endoscope of FIG. 1;
FIG. 9 is a partial cross-sectional view of the endoscope of FIG. 1, taken along line 9-9 in FIG. 1;
FIG. 10 is a partial cross-sectional view of the endoscope of FIG. 1, taken along line 10-10 in FIG. 1; and
FIG. 11 is a schematic diagram of a system for viewing a visually obscured portion of a cavity.
FIG.12 is a side view of the endoscope as it could be a ureteroscope, cystoscope or a nephroscope, without use limitation in other instruments or systems, with the already described working sheath and gripping handle of the preferred embodiment and the imaging apparatus.
FIG.13 side view of the endoscope of FIG. 12 without the gripping handle cover or housing for the rotor.
FIG.14 aerial view from the upper surface of the same endoscope of FIG. 13 on which the different elastic properties of the working sheath are more clearly shown and its interrelation with the rotor are more graphically demonstrated on which right rotor direction, produces a right deflection of the active flexible section.
FIG. 15 same as FIG.14 with contra lateral or left rotor direction producing left deflection of the active flexible section.
FIG. 16 a shorter luminous conductor alternative is presented which is coupled to a battery powered light source. It avoids the approximately 60 cm long conventional luminous conductor.
Fig. 17 this aerial view of an endoscope depicts the alternate gripping handle embodiment with two image collectors that render three-Dimensional view.

While the invention will be described in connection with the preferred embodiment, it will be understood that it is not intended to limit the invention to that embodiment. On the contrary, it is intended to cover all alternatives, modifications, and equivalents, as may be included within the spirit and scope of the invention as defined by the appended claims.

### DETAILED DESCRIPTION OF THE PREFERRED EMBODIMENT

The present invention will now be described more fully hereinafter with reference lo the accompanying drawings which illustrate embodiments of the invention. This invention may, however, be embodied in many different forms and should not be construed as limited lo the illustrated embodiments set forth herein. Rather, these embodiments are provided so that this disclosure will be thorough and complete, and will fully convey the scope of the invention to those skilled in the art. Like reference numbers refer to like elements throughout, and the prime notation, if used, indicates similar elements In alterative embodiments. The preferred embodiment of the present invention implements an endoscopic type instrument, or endoscope, which may be in the form of a ureteroscope.

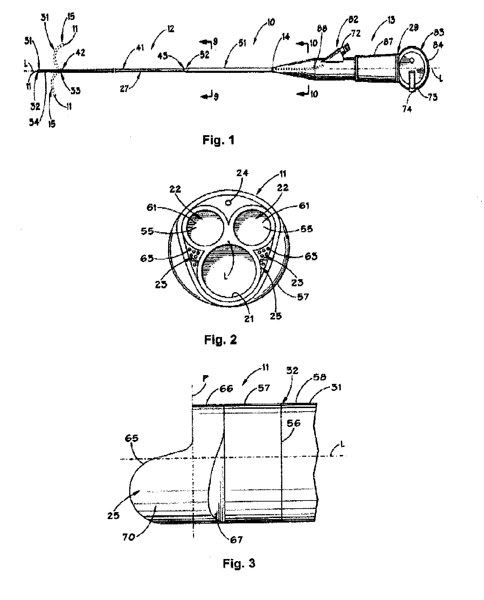

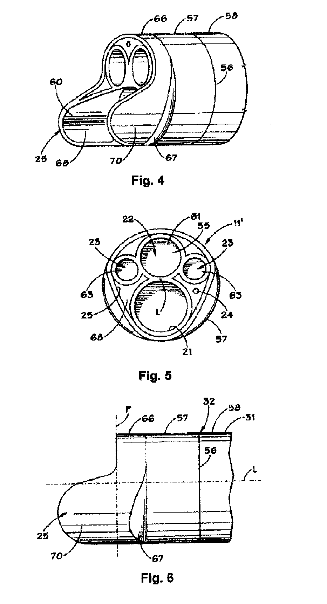

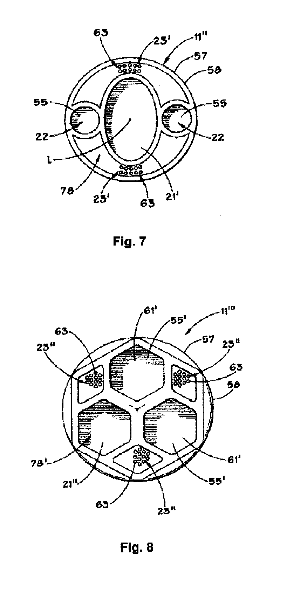

Referring now to the drawings, a first embodiment of the present invention in the form of a ureteroscope 10 is illustrated in FIGS. 1-4. This ureteroscope 10 is only one of many variations of endoscopes, of endoscopic type instruments, that can be produced using the teachings of the present invention. The preferred embodiment of a ureteroscope 10 of the present invention generally comprises: a face tip assembly 11 connected to a working sheath, or working sheath assembly 12, the working sheath assembly 12 being connected to, or associated with, a gripping handle and viewing assembly 13. In conjunction with the face tip assembly 11, the working sheath assembly 12 provides for a reduced risk of laceration of a cavity by allowing the use, or viewing, of conventional, instrument accessories, or "operating tools" (not shown), by providing a tool exit, or port 21, in front of an optical image collector 61, FIGS. 2 and 5, of an exit, or port 21', in the center of the face tip assembly 11, FIG. 7. The working sheath assembly 12 also: provides for simultaneous usage of both the viewing apparatus and an operating tool; facilitates exploration in such cavities as the upper urinary tract; and avoids the necessity for excessive rotation of instruments when targeting, or viewing, is required while working inside such sensitive cavities such as the ureter.

Referring now to FIGS. 2-4, an embodiment of face tip assembly 11 includes a plurality of input/output ports. The ports may include: an operating tool port, or tool exit, 21 where conventional operating accessories (not shown) may exit and are introduced; at least one optical image channel port 22, and at least one luminous channel port 23. The face tip assembly 11 may also include at least one fluid and/or suction channel port 24. The face tip assembly 11 also includes at least one optical image collector 61 interfaced with the al least one optical image channel port 22 of face tip assembly 11, for gathering an image from within the interior body cavity. The type of optical image collector 61 corresponds with the type of optical conductor 62 utilized in ureteroscope 10. For example, selection of an optical waveguide to implement the optical conductor 62 may result in the requirement for a lens, or prism, as an optical image collector 61. If the means for implementing the optical conductor 62 utilized is fiber optics, such as a fiber optics bundle or array, the face of the fiber-optics array may, in turn, be the only means required lo collect the optical image for transmission through the optical conductor 62 through lo the gripping handle and viewing assembly 13, albeit, with reduced visual acuity. The face tip assembly design, of FIGS. 2-4, a" well as other designs hereinafter described, provide the mobility to access the upper urinary system within the kidney and incorporate improved visibility so as to avoid the "blind spot" inherent in many systems comprising the state-of -the-art.

A preferred embodiment of the face tip assembly 11 is best shown in FIGS. 2-4, as a three-dimensional viewing face tip design. In this embodiment, the face tip assembly 11 is a separate unit associated with, or connected to, the distal end 32 of a first flexible working sheath segment 31 of working sheath assembly 12. In this embodiment, where the distal end 32 has a substantially circular cross-sectional shape, the face tip assembly 11 is "face shaped", or appears as having two eyes and a mouth, as shown in FIG. 2. The face tip assembly 11 includes a plurality of optical image collectors 61, which, in this embodiment take the form of a pair of lenses 55 which provide for a three-dimensional view.

The lenses 55 are preferably positioned in a plane P disposed substantially perpendicular to the longitudinal L axis of working sheath segment 31 and working sheath assembly 12 and face assembly 11 (as shown in FIG. 3). Plane P may also be considered to be disposed substantially parallel to the interface 56 between face tip assembly 11 and the distal end 32 of flexible segment 31 of working sheath assembly 12. As illustrated, the two lenses are preferably spaced upwardly of the longitudinal axis L. In this embodiment, face tip assembly 11 also includes an operating tool port 21 which is preferably offset from the center of the face tip assembly 11, or longitudinal axis L, away from the lenses 55, toward the outer perimeter 57, of face tip assembly 11. In this embodiment, the face tip assembly's 11 outer perimeter 57 may be partially congruent with the outer perimeter 58 of the distal end 32 of the first flexible working sheath segment 31. In this embodiment, luminous conductors 63 for light conduction and illumination, in the form of a fiber optics bundle, array, or a single fiber optic strand is located in a portion of the spaces between the lenses 55 and operating tool port 21, as shown in FIG. 2

As shown in FIGS. 3 and 4, face tip assembly 11 may have a working channel port extension 25 which may be in the form of at least one protrusion 65 which functions as an operating tool guide that tends to restrict movement of the operating tool to movement generally along longitudinal axis L. As an operating tool (not shown) exits from operating tool, or tool, port 21, the protrusion 65 may act as a guide lo prevent the operating tool from moving outwardly toward the adjacent wall surface of an adjacent body passageway (not shown) until after the end of tool is visible to the operator via lenses 55. Additionally, protrusion 65 provides protection of the lenses 55 from impact with particulate matter (stone fragments, etc.). The protrusion 65 may be formed integrally as a unitary structure with the outer perimeter 57 of face lip assembly 11. The protrusion 65 may have two spaced apart peaks 60, 70 extending forwardly toward a distal end of the face tip assembly and from a smooth rounded outer distal surface 66. The peaks 60, 70 may preferably be disposed adjacent each side of the tool port 21, as shown in FIGS. 2-4. In this embodiment, where the protrusion 65 is formed as a unitary structure, the protrusion 65 has a concave proximal deflection 67 adjacent outer perimeter 57 in a spaced relationship from interface 56. Alternatively, the working channel port extension 25 may be a separate structure connected to the front of face tip assembly 11. Although the working channel port extension 25 shown in FIGS. 3-4 is a unitary structure with a smooth rounded outer distal surface 66 and a smooth inner surface 68 smoothly contoured and tapering toward operating tool port 21, one of ordinary skill in the art would understand there are many variations of positioning the working channel port extension 25 within the spirit and scope of the present invention. Preferably, as shown in FIGS. 3, 4, and 6, the peaks 60, 70 are disposed offset from the center of the face lip assemblies 11, 11', or longitudinal axis L, and are disposed with a substantial portion of peaks 60, 70 disposed below longitudinal axis L.

Additionally, although the operating tool port 21, lenses 55, and the distal end 32 of first flexible segment 31 are depicted as having generally, circular cross-sectional configurations for the preferred embodiment, it is important to note that in variations of this embodiment, other geometric shapes as known by those of ordinary skill in the art, are within the spirit of the disclosure, such as elliptical, oval, or other shapes. Also, still within the spirit of the preferred embodiment, the distal end 32 of first flexible segment 31 may have a smaller circumference, or diameter, than the main working sheath body 34 of first flexible working sheath segment 31, whereby the outer perimeter 57 of face tip assembly 11 may be al least partially received around, and connected to, the smaller outer circumference of distal end 32. Still referring to FIGS. 2-4, face tip assembly 11, may include variations in the shape of outer perimeter 57, variations in the positioning, or location, of lenses 55, operating tool port 21, and optical conductor 62. Additionally, in other embodiments of the face tip assembly 11 structure depicted in FIG. 24, the optical image collector 61 may be in another form such as prisms or a substantially flush bundle of fiber optics or other methodologies as known by those of ordinary skill in the art. The optical conductor 62 and luminous conductors 63 may also be in any acceptable form as known by those of ordinary skill in the art that can perform substantially the same function as fiber optics or as the microscopic frontal camera at the face tip assembly to gather images and it is laced a the distal end of endoscopic type instruments.

Referring now to FIGS. 5-6, a monoptic face-tip assembly embodiment is illustrated. In this face tip assembly 11', the general shape of the outer circumference 57 and protrusion 65 are substantially similar to those described in connection with FIGS. 2-4. In this embodiment, the face tip assembly 11' is a separate unit connected to the distal end 32 of first flexible working sheath segment 31. In this embodiment, when the distal end 32 has a substantially circular cross-sectional configuration, the face tip assembly 11' may have a corresponding generally, circular cross-sectional configuration shown in FIG. 5. The face tip assembly 11 includes a single optical image collector 61, which, in this embodiment takes the form of a single lens 55 which provides for a view lo help those users with difficulty in adapting to three-dimensional viewing. The lens 55 is positioned, or disposed, on plane P and spaced from longitudinal axis L, and offset toward the outer perimeter 57. In this embodiment, the face tip assembly 11' also includes an operating tool port 21 which is offset from the center, or longitudinal axis L, of the face tip assembly 11', away from the lens 55, toward the outer perimeter 57 opposite that of lens 55. In this embodiment, outer perimeter 57 of face tip assembly 11', is partially congruent with the outer perimeter 58 of the distal end 32 of the first flexible segment 31. Luminous conductors 63 for light conduction and illumination in the form of fiber optics may be located on opposite sides of the lens 55. In this embodiment, face tip assembly 11' also has a working channel port extension 25 in the form of the protrusion 65, previously described, which may function as an operating tool guide as previously described, and provides protection of the lens 55 from impact with particulate matter (stone fragments, etc.). In this embodiment, the protrusion 65 is again formed integrally as a unitary structure with the outer perimeter 57 of face tip assembly 11'. The protrusion 65 has two peaks 69, 70, formed by smooth rounded outer distal surface 66. In this embodiment, when the protrusion 65 is formed of a unitary structure, the protrusion 65 also may have a concave proximal deflection 67, spaced from interface 56. The working channel port extension 25 may be a separate structure connected to the front of face tip assembly 11'.

Although the working channel port extension 25 is shown in FIGS. 5-6 as a unitary structure extending forwardly from a smooth rounded outer distal surface 66 and a smooth inner surface 68 smoothly contoured and tapering toward operating tool port 21. One of ordinary skill in the art would understand there are many variations of positioning the working channel port extension 25 within the spirit of the disclosure. Additionally, although the operating tool port 21, lens 55, and the distal end 32 of first flexible working sheath segment 31 are depicted as having generally circular shapes for this embodiment, in variations of this embodiment, other geometric designs, or shapes, as known by those of ordinary skill in the art, are within the scope of the present invention. Again, the distal end 32 of first flexible segment 31 may have a smaller circumference than the main working sheath body 34 of first flexible working sheath segment 31, whereby the outer perimeter 57 of face tip assembly 11 may be at least partially received around, and connected to the smaller outer circumference of distal end 32. Still referring to FIGS. 5-6, there may be variations in the shape of outer perimeter 57, variations in the positioning of lens 55, operating tool port 21, and optical conductors 62. Again, the optical image collector 61 may be in another form such as prisms or a substantially flush bundle of fiber optics or other methodologies as known by those of ordinary skill in the art. The optical conductor 62 and luminous conductors 63 may also be in any acceptable form as known by those of ordinary skill in the art that can perform substantially the same function as fiber optics. The protrusion 65 in the embodiments discussed regarding FIGS. 2-4 and FIGS. 5-6 may be in more of a form similar to a semicircular hollow cylinder of a more equal distal height as opposed to a form similar to peaks and valleys as described above, as well as may have the other shapes which provide the desired tool guiding functions.

With reference to FIG. 7, an alternative three- dimensional viewing face-tip assembly 11" is shown. The face tip assembly 11", generally has the shape of the face of face tip assemblies 11 and 11', but is generally flatter in appearance, and lacks substantial protrusions 65. In other words, the front face 78 of face tip assembly 11" generally lies in a plane substantially parallel with plane P previously described. The face tip assembly 11" may also include a generally elliptical shaped operating tool port 21' which is substantially centered between the lenses 55. Disposed on either side of port 21' plurality of optical image collectors 61, which, in this embodiment may take the form of a pair of lenses 55 which provide for a three-dimensional view. The lenses 55 are generally positioned on a plane substantially parallel with plane P previously described.

Still with reference to FIG. 7, the outer perimeter 57 of face tip assembly 11" is generally congruent with the outer perimeter 58 of the distal end 32 of the first flexible working sheath segment 31. In this embodiment, luminous conductors 63 for light conduction and illumination in the form of fiber optics as previously described, may be positioned above and below the elliptical shaped port 21'. Although the operating tool port 21' is depicted as generally elliptical, and lenses 55 and the distal end 32 off first flexible segment 31 are depicted as circular for this embodiment, it is important to note that in variations of this embodiment, other geometric designs and shapes, as known by those of ordinary skill in the art, are within the scope of the present invention. Also, still within the spirit of this embodiment, the distal end 32 of first flexible working sheath segment 31 may have a smaller circumference than the main working sheath body 34 of first flexible working sheath segment 31, whereby the outer perimeter 57 of face tip assembly 11 may be at least partially received around, and connected to, the smaller outer circumference of distal end 32 Still referring to FIG. 7, another embodiment may include variations in the shape of outer perimeter 57, variations in the positioning of lenses 55, operating tool port 21', and optical conductor 63. Additionally, in other embodiments of the face tip assembly 11' structure depicted in FIG. 7, the optical image collector 61 may be in another form such as prisms or a substantially flush bundle of fiber optics or other methodologies as known by those of ordinary skill in the art. The optical conductor 62 and luminous conductors 63 may also be in any acceptable form as known by those of ordinary skill in the art that can perform substantially the same function as fiber optics.

Referring now lo FIG. 8, another alternative three-dimensional viewing face-tip assembly 11"' is shown, In this face tip assembly 11"', the general shape of the face 78' of face tip assembly 11"' is flatter in appearance than those described in FIGS. 2-4 and FIGS. 5-6 and thus lacks a substantial protrusion 25 formed by peaks 69, 70. Front face 78' also generally lies in a plane substantially parallel with plane P previously described. The face tip assembly 11"' may include a pair of optical image collectors 61', which, in this; embodiment take the form of a plurality of hexagonal shaped lenses 55' which provide for a three-dimensional view. The lenses 55' are positioned in plane P as previously described and are offset toward the outer perimeter 57. In this embodiment, the face tip assembly 11"' also includes an operating tool port 21" which is offset from the center, or longitudinal axis L, of the face tip assembly 11"', away from the lenses 55', toward the outer perimeter 57. In this embodiment, luminous conductors 63 for light conduction and illumination in the form of fiber optics are disposed in luminous channel ports 23" located circumferentially between the lenses 55' and between lenses 55' and operating tool port 21" and form a generally triangular shaped array wherein each port 23" is located al the tips of the triangle. In this embodiment, face tip assembly 11 has a hexagonal shape and connects with, or alternatively is a part of, the first flexible working sheath segment 31. Although the operating tool port 21" and lenses 55' are hexagonal shaped, luminous channel ports 23" is diamond shaped, and the distal end 32 of first flexible working sheath segment 31 is depicted as circular, it is important lo note that variations of this embodiment, would permit other geometric designs as known by those of ordinary skill in the art, within the scope of the invention. Also, the distal end 32 of first flexible working sheath segment 31 may be in the shape of a hexagon and have a smaller circumference than the main body 34 of first flexible working sheath segment 31 whereby the outer perimeter 57 of face tip assembly 11"' may be at least partially received around, and Connected to, the smaller outer perimeter of distal end 32. Still referring to FIG. 8, another embodiment may include variations in the shape of outer perimeter 57, variations in the positioning of lenses 55", operating tool port 21", and fiber-optic optical conductor 62. Additionally, in other embodiments of the face tip assembly 11 "' depicted in FIG. 8, the optical image collector 61' may be in another form such as a prism or a substantially flush bundle of fiber optics or other methodologies as known by those of ordinary skill in the art. The optical conductor 62 and luminous conductors 63 may also be in any acceptable form as known by those of ordinary skill in the art that can perform substantially the same function as fiber optics.

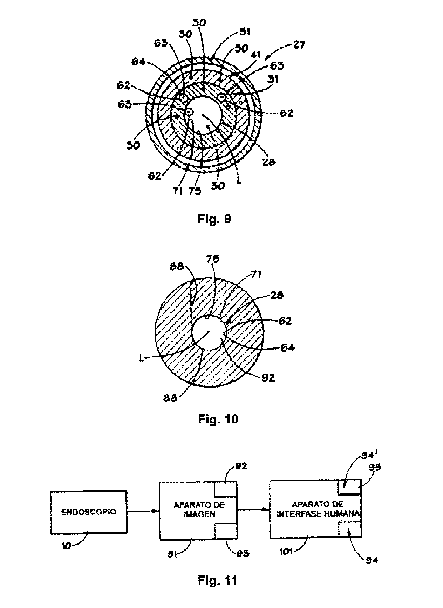

Referring to FIGS. 1 and 9, the working sheath assembly 12 of ureteroscope, or endoscope, 10 includes a working sheath 27 having at least one longitudinally extending passageway 28 and gripping handle and viewing assembly interface 29. Preferably, there is a passageway 28, which corresponds to, and is in communication with, each operating tool port 21-21", optical image channel port 22, 22', and luminous channel port 22-23". The working sheath 27 is preferably constructed of a suitable nontoxic material, such as a plastic or polymer material and includes a first flexible working sheath segment 31 having distal end 32 adapted for insertion into the cavity and interfaced with the face tip assembly 11 at interface 56; a second flexible working sheath segment 41 having a distal end 42 connected to a proximal end 33 of the first flexible working sheath segment 31; and a third working sheath segment 51 having a distal end 52 connected 10 a proximal end 43 of the second flexible working sheath segment 41.

Preferably, the working sheath 27 is constructed so that it has a substantially smooth, continuous outer surface, and its preferred cross-sectional configuration is circular. Preferably the length of the third working sheath segment 51 is approximately 50 cm long The first flexible working sheath section 31 is preferably approximately 4 cm long, and the second flexible working sheath section 41 is preferably approximately 20 cm long. The first and second flexible working sheath sections 31,41 preferably have cross-sectional configurations that are substantially uniform along their lengths, but they may taper downwardly toward the face tip assembly 11. The third section 51 of working sheath 27 is constructed so that it has sufficient strength and rigidity to permit use within the bladder and to support the entry of the first and second flexible sections 31, 41 into the ureter and may be described as rigid or semi-rigid in construction. The first and second flexible working sheath sections 31, 41 are constructed in order to follow the contours of the ureter. Also, as is known by those of ordinary skill in the art of endoscopes, the lengths of the first segment 31, second segment 41, and third segment 51 of the working sheath 27 may vary according to the intended use of the endoscope 10.

The third working sheath segment 51 is dimensioned to be received in a human body so that it extends through the urethra and substantially through the bladder, The distal end 52 of segment 51 is tapered to receive the proximal end 43 of the second flexible segment 41 and is formed 10 provide a smooth, gradual transition between the second flexible segment 41 and the third segment 51, to permit the nontraumatic passage of the working sheath 27 through the urethra and into the bladder. Preferably, the third section 51, preferably, has sufficient strength and rigidity lo enable both axial and rotational translation with the maneuvering of the gripping handle and viewing assembly 13, without excessive twisting of the working sheath 27. Additionally, the connection 14 between working sheath segment 51 and the gripping handle and viewing assembly 13 bas sufficient strength and rigidity to avoid breaking during use and handling of endoscope 10. Thus, the user is able to insert the working sheath 27, leading with face tip assembly 11, into the urethra and maneuver the instrument through the bladder in order to position the first flexible section 31 and thus the face tip assembly 11 into the opening of the ureter. The first flexible segment 31 having distal end 32 adapted for insertion into the cavity is dimensioned to be received in the ureter of a patient.

The second flexible working sheath segment 41 having a distal end 42, like first flexible working sheath 31 is correspondingly also dimensioned lo be received in the ureter of a patient and is sufficiently flexible along its length to follow various canals of the human body, such as the ureter. In order to optimize the versatility of a flexible endoscope while retaining the controllability of a rigid endoscope, the second flexible segment 41 is "passively flexible". The term "passively flexible" is intended to mean that working sheath segment 41 may be moved, flexed, or bent, to assume a curved configuration, in response to forces exerted upon the working sheath 27 as it passes through a cavity or body passageway, but the movement, flexing, or bending is not substantially controllable by the operator of the instrument. While the third working sheath segment 51 provides the user with sufficient feel and control of the instrument 10, the second flexible segment 41 has the ability to readily flex and follow the contours of a cavity or passageway, such as the ureter, without excessive deformation of its cavity or passageway, in order to minimize any traumatic effects.

In contrast, the first flexible working sheath segment 31 is "actively flexible". The term "actively flexible" is intended lo mean that working sheath segment 31 may be moved, flexed, or bent to assume a curved configuration, such as shown in phantom lines 15 in FIG. 1, or an angular disposition with respect to longitudinal axis L, and such movement, flexing or bending is substantially controlled by the operator, who can cause and control the desired movement, flexing, and/or bending. The deflection of face tip assembly 11 upon operator, or user, command, or control, aids the user in the detection and penetration of the opening of the ureter. Additionally, the relatively small diameters of face tip assembly 11 and first flexible working sheath segment 31 allow the user to insert the working sheath 27 into the narrow opening of the ureter lo gain access to the ureter and kidney. The active flexibility of the first flexible working sheath segment 31 also provides for nontraumatic use of the instrument 10 and precise positioning of the face tip assembly 11 adjacent to items of interest such as a lesion or kidney stone. Most significantly, the actively flexible first flexible segment 31 enables the user to view and, along with other features of the present invention, non-traumatically deliver a working tool via the working channel 71 and operating tool port 21 to the item of interest. The flexibility of the first flexible segment 31 generally negates the need for rotating the instrument when targeting or advancing the instrument as required.

The first flexible working sheath segment 31 may be made actively flexible using various methodologies. In the preferred embodiment, the first flexible segment 31 is made actively flexible through use of operating, or guide, wires 30 guided through individual conduits; which pass longitudinally through working sheath segment 31 or through passageway 28 within working sheath 27 toward the distal end 32, which wire, or wires, may be manipulated, or pulled, so as lo bend, move, or flex, the working sheath segment 31 in a desired direction. The distal ends of the wires 30 may be suitably anchored adjacent he distal end 32 of working sheath segment 31, whereby upon pulling on the wire, or wires 30, the desired controlled flexing, moving, or bending will occur. Alternatively, the first flexible working sheath segment 31 may be comprised of a connected string of body members consisting of semicircular disc-like ring elements forming selectively controllable expandable bodies, whereby upon controlled expansion of selected ring elements, the working sheath segment 31 moves or flexes in the desired direction, similar to the manner in which a snake moves. Other methodologies for providing the requisite flexibility could include the use of springs, separate wire guides, or the working tool itself, among others. If desired, the cross-sectional shape of first flexible working sheath segment 31 could be varied in order lo provide varying inherent flexibility characteristics. In other words, one or more portions, or sides, of the first flexible working sheath segment 31 can be made to be more pliable, or flexible, than other portions, or sides, of the same first flexible working sheath segment in order lo make a working sheath segment that more readily flexes in a first direction and is more rigid in a second direction.

Alternatively, the first flexible working sheath segment 31 can be made from a composite material that has differing properties that will result in having a first flexible segment 31 predisposed lo more readily bend, or flex, in a first direction, for example, upwardly and downwardly, rather than from side to side. Alternatively, the active desired flexibility of the first flexible working sheath segment 31 could be obtained by use of a longitudinally disposed tension cable with a distal spring deflection recovery member, whereby increased tension or compression on the tension cable initialed through a suitable control causes the flexible working sheath segment 31 to deflect or flex in a desired direction.

Face tip assemblies 11, 11', 11", 11"' may be a separate multi -port piece which is connected to the distal end 32 of first flexible working sheath segment 31 of working sheath 27 as previously described. In an alternative embodiment, the face tip assemblies 11-11"' may be a unitary piece formed integral with first flexible segment 31 as previously described. If desired, the same material used lo form the first flexible working sheath segment 31 may also be used for the second flexible working sheath segment 41. The first flexible working sheath segment 31 may have approximately the same diameter as the second flexible working sheath segment 41, and the two segments may be formed integral with each other or formed separately and connected by any suitable connection. If desired, as seen in FIGS. 1 and 9, the first working sheath segment 31 could extend along the longitudinal axis L of the working sheath 27 from its distal end 32 to the gripping handle and viewing assembly, whereby working sheath segment 31 is concentrically disposed within the second working sheath segment 41 and third working sheath segment 51. In turn, the second working sheath segment could also extend along the longitudinal axis L of working sheath 27 lo the gripping handle and viewing assembly 13, whereby working sheath segment 41 is concentrically disposed within the third working sheath segment 51. Where the first working sheath segment 31 enters the second working sheath segment 41, and where the second working sheath segment 41 enters the third working sheath segment 51, define transition zones, or transition locations, 39, 49, and preferably at these zones the larger diameter working sheath segment as shown al zone 49 in FIG. 1. These tapering transition zones 39, 49 provide increased durability of the working sheath 27 to bending fatigue and ease the insertion of the working sheath 27 into the desired body cavity. The second flexible segment 41 may have a different diameter than the third segment 51, and the second flexible segment 41 may be disposed inside the third segment 51. In the preferred embodiment, the first and second flexible section 31,41 have a diameter of approximately 7.2 French equal to approximately 2.16 millimeters, wherein the third segment 51 has a diameter of approximately 8.2 French equal to approximately 2.46 millimeters.

With reference lo FIGS. 1, 9, and 10 the gripping handle and viewing assembly 13 has a plurality of passageways, or channels, 88 in communication with corresponding passageways, or channels, 28 of working sheath 27 and longitudinally extend lo the first flexible working sheath segment 31 lo the input/output ports of the face tip assemblies 11-11"'. The passageways, or channels, between face tip assembly 11 and gripping handle and viewing assembly 13 may be of equal diameter, or of differing diameter sizes, whereby they taper from one end to another to provide a smooth continuation of the passageways or channels.

With reference to FIGS. 1, 9, and 10, the gripping handle and viewing assembly 13 includes: a distal section 81 which connects, or interfaces, with working sheath 27, a working channel interface section 82 including a working channel interface assembly 72 which provides access for various operating tools through the instrument 10; a luminous conductor interface assembly 73 which provides for connecting, or interfacing, a light source such as a lamp box, for example, \with the luminous conductor 63; and a proximal section 83 including proximal section assembly 84, including optical channel interface assembly 74, and which provides either an interface, or an intermediate connection, to a conventional imaging apparatus (not shown). The gripping handle and viewing assembly 13 may include, if desired, any one or more of the following connection components: a handhold or pistol-type grip; a telescopic viewing assembly; an eyepiece adjustment; an optical tap for transmission of the optical image to an imaging apparatus; an electronic image enhancer/transmitter: and or valve(s) for irrigation/suction.

The instrument 10 includes a working channel 71 for providing a pathway into the internal cavity for a conventional working instrument. Referring now to FIGS. 1, 2, 9 and 10, in the preferred embodiment, the working channel 71 is formed via passageways 28, 88 and provides working tool access to the interior cavity, the channel 71 extending from the working channel interface assembly 72 through lo the operating tool port 21. In an embodiment, the working channel 71 has a substantially smooth interior surface to provide smooth movement of a working tool through instrument 10. The working channel 71 may have a substantially circular cross-sectional configuration, and may be coaxially surrounded by working sheath segments 31,41, and 51 of working sheath 27. The interior wall surface 75 of working channel 71 may be coated with, or formed of, a material having a reduced coefficient of friction to facilitate easy passage and use of working accessories, or tools, in the working channel 71.

The instrument 10 includes al least one luminous conductor 63 for providing illumination within the interior cavity. The luminous conductor 63 extends from the luminous conductor interface assembly 73 of gripping handle and viewing assembly 13, through working sheath 27, to distal end 32 of first flexible working sheath segment 31 to face assembly 11-11"'. The luminous conductor 63 is in the form of a fiber optic light carrying bundle. The luminous conductor interface assembly 73 provides a connector, as understood by those skilled in the art, between the luminous conductor 63 (light guide) and a conventional light source (not shown) Light travels through the luminous conductor interface assembly 73 and through the gripping handle and viewing assembly housing 87 and working sheath 27 to the interior cavity in a manner depending upon the configuration of the face tip assembly 11-11"'. For example, in one of the embodiments described with regard lo FIG. 2, the luminous conductor 63 is a single fiber-optic bundle and may be interspersed among the optical conductor 62 in working channel 71. However, in one of the embodiments of FIGS. 3, 4, or 5, the implementation may be best had through a plurality of independent fiber-optic bundles or a single fiber-optic bundle divided prior to or upon reaching luminous channel port 23. Also, in an embodiment, the luminous conductor interface assembly 73 may include an adjustable light valve (not shown) for selectively adjusting the intensity of the light. In another embodiment, the gripping handle and viewing assembly 13 may include a plurality of the luminous conductor interface assemblies 73.

Referring again to FIGS. 1 and 9, an embodiment of the present invention also comprises at least one optical conductor 62 optically interfaced with the optical collector 61 for transmitting the gathered interior cavity image to the gripping handle and viewing assembly 13. In the preferred embodiment, the optical conductor 62 is in the form of a fiber-optic bundle 64. In this embodiment, the instrument 10 includes an optical conductor channel 92 which encloses and receives the optical conductor 82, 64. The optical conductor 62, 64 may be located within the instrument 10, such as by disposing it in the working channel 71, or it may be formed as a separate channel. A luminous conductor channel 93 may be provided to carry light to the face tip assembly 11-11"' and correspondingly the internal cavity and thus, the area of interest. A fused fiber optic image bundle 62 would extend through working sheath 27 lo the face tip assembly 11-11"' and correspondingly to the optical image collector 61. In an embodiment, the optical conductor 62 is supported within gripping handle and viewing assembly housing 87 by means known by those skilled in the art. For example, the optical conductor 62 would be supported within the gripping handle and viewing assembly housing 87. The gripping handle and viewing assembly 13 of endoscope 10 may be equipped to interface with an imaging apparatus 91 (FIG. 11) having an imaging processor 93 in order to capture the image gathered by optical image collector 61 in order lo process the image for transmission to a human interface apparatus 101, such as a monitor and/or lo video capable glasses. In an alternative embodiment, the gripping handle and viewing assembly 13 is used as a form of telescope as known by those skilled in the art, whereby an ocular lens and lens support would cooperate with a spring means to permit relative movement between the optical conductor 62 and the housing 87 lo provide direct, adjustable, visual imaging. In an embodiment, an optical wedge (not shown) is included, the optical wedge can be located near the distal end 32 of the first flexible segment 31 to provide a direction of view compensation of about 5-10 degrees when viewed under water, as would be the case if implemented as a ureteroscope.

Referring now to FIGS. 1,2, and 11, a system to view a visually obscured portion of a body cavity will be described. The system may include an endoscope 10 as previously described. The system may further include an imaging apparatus 91 coupled with the at least one optical conductor 62 via the gripping handle and viewing assembly 13, for capturing the image to send it to a human interface apparatus 101. In an embodiment, instead of the user strictly viewing the image gathered by the optical image collector 61 through a telescope or eye piece portion of a viewing assembly as is the case with much of the state-of-the-art, the gripping handle and viewing assembly 13 of the present invention may include a proximal section assembly 84 which provides an interface for the imaging apparatus 91 as known by those skilled in the art. In an embodiment, the imaging apparatus 91 is an image transceiver 92 including an image processor 93 capable of providing video output lo a human interface apparatus 101. In another embodiment, the imaging apparatus 91 is a pair of cameras optically coupled with a plurality of optical conductors 62. The preferred function of the imaging apparatus 91 is to render a three-dimensional image of the area of interest as selected by the user. Typically this is accomplished using individual "optical feeds." Additionally, in the preferred embodiment utilizing a pair of optical conductors 62 and optical image collectors 61, the imaging apparatus 91 captures each half of the image to render a complete and broader view of the area of interest.

The system may include a human-interface apparatus 101, as shown in FIG. 11. The human interface apparatus 101 is electrically or optically coupled with the imaging apparatus 91. In various embodiments, the human interface apparatus 101 may include such display/interface devices including a first image display device 94 such as a CRT, HDTV, for example, and in the preferred embodiment, a second image display device 94 including a video stereoscopic viewer unit 95 as known and understood by those skilled in the art. Although visual clarity is an important feature of the human interface apparatus 101, the invention is not limited to, or to the quality of, the examples provided above.

An embodiment of the present invention includes a method of performing a procedure in a visually obscured portion of a body cavity while under direct visual control. Specifically, the method of the present invention comprises the steps of: providing an endoscopic type instrument 10, having a face tip assembly, such as face tip assembly 11-11"' connected lo a working sheath assembly 12 having an actively flexible working sheath segment 31 at its distal end, the working sheath assembly 12 being connected to a gripping handle and viewing assembly 13; providing an illumination source, such as luminous conductor 62; inserting the distal end of the working sheath assembly into a body cavity; manipulating the actively flexible working sheath segment to a desired angular deflection in order to properly target, or view, the area of interest to allow both diagnosis and operative procedures. Another step may be advancing a working tool through the working channel 71, into the body cavity, while simultaneously monitoring its exit through the face tip assembly 11-11"'. The user can view the inner portion of a cavity such as, for example, the ureter or kidneys, and simultaneously view the insertion of a working/operating tool. Thus, various procedures can be carried out within the cavity while under direct visual control. The method may include the steps of irrigating the area of interest and suctioning particulate matter from the body cavity. Note, that one skilled in the art would know that some of the above steps do not need lo be accomplished in the order provided in this embodiment. The method may also include the step of viewing during insertion, the relative location of the face tip assembly to properly position the assembly with respect to an area of interest 114;

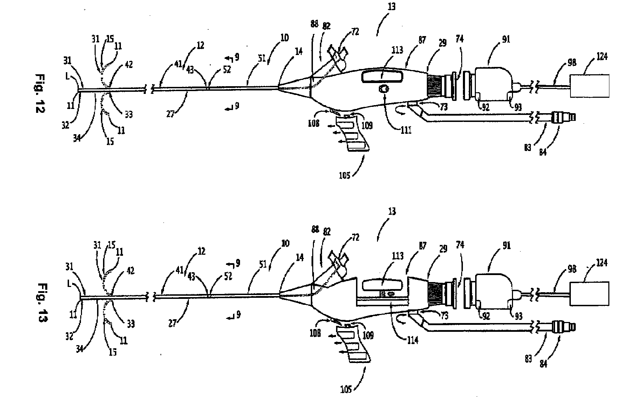

Referring now to Fig. 12-16 a first embodiment of the present invention is in the form of a ureteroscope 10. This ureteroscope 10 may however be embodied in many different forms of endoscopes or endoscope-Type instruments that can be manufactured with the teaching of the present invention. The preferred embodiment of ureteroscope 10 comprising a working sheath of different elastic properties 12, which is joined or united at gripping handle 13.

Referring now to FIG. 12-13, a lateral view of the endoscope system in which will be described a housing 87 for al gripping handle elements 13 with a fluid design in accordance to the working sheath concept of multiple elasticities or elastic properties, it incorporates a rotor 113 as a circular shape like a wheel; being this geometric form or any other shape, dimension, height or space in the three Cartesian planes a limitation. The rotor 113 through different operational methodologies provides action of deflection to first active flexible segment 31 through wires 30 or guiding cables through longitudinal individual conduits along working sheath 12 coming from holding wires elements at the base of rotor 114.

Still in reference to FIG. 12 at the proximal end of the gripping handle; a luminous conductor interface 73 is found at the initial third and below the longitudinal axis L, and the accompanying arrows in front of it reflect that the luminous conductor interface 73 and its elements; the luminous conductor 83 and the proximal section assembly of the luminous conductor 84 can rotate 180° to each side. All luminous elements described 73,83 and 84 are referred in this embodiment in such a manner that cable 98 of the imaging apparatus 91 that usually measures 60 cm or more in length connects wit an imaging processor 124 and during handling is maintained along the longitudinal axis L of endoscope 10 and in synchrony with the working sheath assembly of different elastic properties in such a manner that when turning or rotating of the instrument is required in the x axis of the Cartesian planes, these three elements will move harmonically and in parallel, thus preventing encountered, non linear, parallel or coordinated vectorial forces. This improves endoscope 10 control.

Still in reference to FIG. 12-13, an stabilizer 105 is provided in gripping handle 13 at its distal third and below the longitudinal axis L, along gripping handle 13 in a gripping handle shape or any other form to maintain the stability of endoscope 10 as long as necessary. The stabilizer 105 is provided with a solid union attachment device 109 at its superior area to be joined to a solid coupler accessory 108 found at gripping handle 13. The stabilizer 105 is an optional accessory, depending upon where the endoscope 10 is being utilized.

In reference to FIG. 12 it is shown the brake mechanism 111 at gripping handle 13 at its left side; it is employed to maintain in a certain position the active flexible segment 31 when an area of interest has been identified, should the operator requires to observe another area than the operating field during the procedure. This brake 111 fixates the active flexible segment 31; however rotor 113 could be moved as a gear mechanism (not shown) and step by step during rotor motion, change position of the active flexible segment 31.

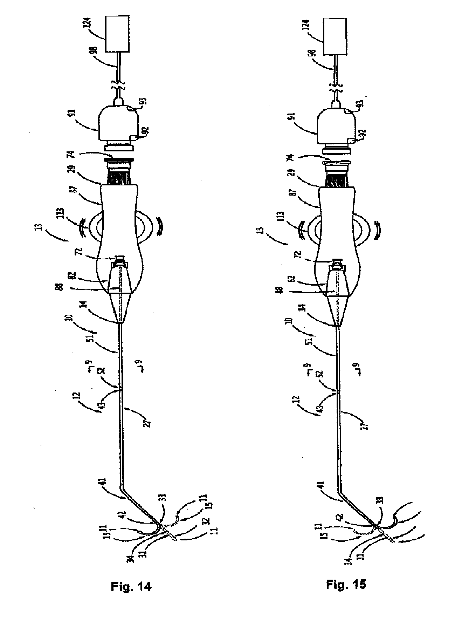

In reference to FIG. 14 it is shown intuitivness of endoscope 10. This aerial view at instrument's resting position shows that when rotor 113 turns to the right, in a synchronous and simultaneous manner turns to the right also, the active flexible segment 31 of working sheath 27.

In reference to FIG. 15 it is shown intuitivness of endoscope 10. This aerial view at instruments resting position shows that when rotor 113 turns to the left, in a synchronous and simultaneous manner turns to the left also, the active flexible segment 31 of working sheath 27. The second flexible segment 41 is "passively flexible". The term "passively flexible" is intended to mean that working sheath segment 41 may be moved, flexed, or bent, to assume a curved configuration, in response to forces exerted upon the working sheath 27 as it passes through a cavity or body passageway, but the movement, flexing, or bending is not substantially controllable by the operator of the instrument.

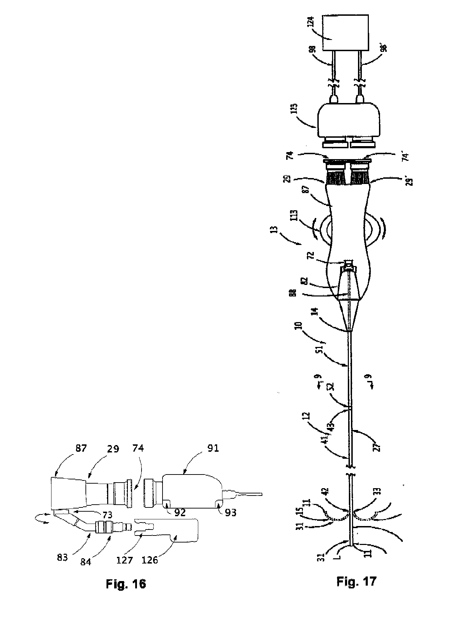

In regard to FIG. 16, the assembly of the luminous conductor interface 73, the proximal section of luminous conductor 83 and proximal section of luminous conductor 84 are a lot shorter, in resemblance of a post that hardly goes over the edge of optical eyepiece 74 and can receive or couple with a battery lamp of any kind 126 with a coupler 127 and nourish light into the obscured cavity of the body; this is instead the long luminous conductor 83 that is typically 60 cm or more in length.

In reference to FIG. 17 is shown that the proximal end of gripping handle 13 has two image collectors 74, 74', coupled to a three dimensional image processor 123 coupled to its cables 98,98' that are joined at an imaging processor or apparatus 124.

In the drawings and specification, there have been disclosed a typical preferred embodiment of the invention, and although specific terms are employed, the terms are used in a descriptive sense only and not for purposes of limitation. The invention has been described in considerable detail with specific reference lo these illustrated embodiments. It will be apparent, however, that various modifications and changes can be made within the spirit and scope of the invention as described in the foregoing specification. It is understood that other materials and dimensions may be used for the endoscopic type instrument of the present invention keeping in mind the dimensions of the affected body parts. Further, the number and dimensions of the channels or passageways employed are variable depending on the accessories (i.e. dye laser, fiber optics, etc.) used in conjunction with the instrument. Additionally, the actively flexible working sheath segment could be used with other working sheath segments which are all rigid, all semi-rigid, all flexible, or combinations thereof. Further, the face tip assemblies may be used with any type of endoscopic instrument or working sheath assembly Also, other shaped gripping handles and gripping handles of other designs may be used. Accordingly, the invention is to be limited only by the scope of the appended claims.

## Claims

1. An endoscope system for viewing a visually obscured portion of a body cavity comprising:
an endoscope having,
a face tip assembly, having a plurality of input/output ports associated with a working sheath assembly, the working sheath assembly being associated with a gripping handle and viewing assembly;
the working sheath assembly including a working sheath having a distal end and an actively flexible working sheath segment disposed at the distal end of the working sheath for insertion into the cavity;
at least one optical image collector adapted to gather an image from within the body cavity;
at least one optical conductor, associated with the at least one optical image collector, and adapted to transmit the image to the gripping handle and viewing assembly;
at least one luminous conductor adapted to provide illumination to the body cavity;
at least one working channel disposed within the working sheath assembly adapted to permit a working instrument entry into the body cavity; and
an imaging apparatus, associated with the at least one optical conductor, and adapted lo capture the image to send it to a human interface apparatus adapted to permit viewing of the image.

2. The endoscope system of claim 1, including a working channel extension, associated with the face tip assembly, which includes at least one protrusion adapted lo guide the working instrument and lo prevent impact of foreign matter upon the al least one optical image collector.

3. The endoscope system of claim 1 wherein there are two optical conductors for producing a three-dimensional image.

4. The endoscope system of claim 3, including an image processor capable of capturing the image to render it three-dimensional for display by the human interface apparatus.

5. The endoscope system of claim 1 wherein there are two image collectors for producing a three-dimensional image.

6. The endoscope system of claim 1, wherein the actively flexible working sheath segment is disposed adjacent a passively flexible working sheath segment.

7. The endoscope system of claim 1, wherein the passively flexible working sheath segment is disposed adjacent a semirigid working sheath segment.

8. An endoscope for viewing a portion of a body cavity, comprising:
a face tip assembly, having a plurality of input/output ports, associated with a working sheath assembly, the working sheath assembly being associated with a gripping handle and viewing assembly;
the working sheath assembly including a longitudinal axis, a working sheath having a distal end, and an actively flexible working sheath segment disposed at the distal end of the working sheath for insertion into the body cavity;
at least one optical image collector adapted to gather an image from within the body cavity;
at least one optical conductor, associated with the at least one optical image collector, and adapted to transmit the image to the gripping handle and viewing assembly;
at least one luminous conductor adapted lo provide illumination to the body cavity; and
at least one working channel disposed within the working sheath assembly and adapted to permit a working instrument entry into the body cavity.

9. The endoscope of claim 8, including a working channel extension, associated with the face tip assembly which includes at least one protrusion to guide the working instrument and lo prevent impact of foreign matter upon the at least one optical image collector.

10. The endoscope of claim 9, wherein the at least one optical image collector lies in a first plane which is disposed substantially perpendicular to the longitudinal axis of the working sheath assembly; and the at least one protrusion is disposed at the distal end of the working sheath forward of the first plane in which the at least one optical image collector lies, whereby the at least one optical image collector may view an operating tool passing forwardly beyond the at least one protrusion.

11. The endoscope of claim 10, wherein the plurality of input/output ports include at least one operating tool port, and the operating tool port lies in a second plane which is disposed substantially parallel with the first plane in which the at least one optical collector lies.

12. The endoscope of claim 11, wherein the first plane and the second plane are substantially coplanar.

13. The endoscope of claim 11, wherein the second plane is disposed in a spaced relationship from the first plane, toward the distal end of the working sheath.

14. The endoscope of claim 9, wherein the al least one protrusion has two peaks which extend forwardly toward a distal end of the face tip assembly.

15. The endoscope of claim 14, wherein the plurality of input/output ports include al least one operating tool port, having two sides, and the two peaks are spaced apart from each other, with a peak disposed adjacent each side of the operating tool port.

16. The endoscope of claim 15, wherein the peaks are disposed offset from the longitudinal axis, with a substantial portion of each peak disposed below the longitudinal axis.

17. The endoscope of claim 8, wherein the plurality of input/output ports include at least one operating tool port, at least one optical image channel port, and at least one luminous channel port.

18. The endoscope of claim 8, wherein the face tip assembly includes al least two optical image collectors, each optical image collector being disposed in a first plane which is disposed substantially perpendicular lo the longitudinal axis of the working sheath assembly.

19. The endoscope of claim 8, wherein the actively flexible working sheath segment is disposed adjacent a passively flexible working sheath segment.

20. The endoscope of claim 19, wherein the passively flexible working sheath segment is disposed adjacent a semi-rigid working sheath segment.

21. The endoscope of claim 8, wherein the actively flexible working sheath segment is disposed adjacent a semi-rigid working sheath segment.

22. An endoscope for viewing a portion of a body cavity, comprising:
a face tip assembly, having a plurality of input/output ports, associated with a working sheath assembly, the working sheath assembly being associated with a gripping handle and viewing assembly;
the working sheath assembly including a longitudinal axis, a working sheath having a distal end, and a working sheath segment disposed at the distal end of the working sheath for insertion into the body cavity;
at least two optical image collectors which produce a three dimensional image from within the body cavity;
at least one optical conductor, associated with the at least two optical image collectors, and adapted to transmit the three dimensional image to the gripping handle and viewing assembly;
at least one luminous conductor adapted to provide illumination to the body cavity; and
at least one working channel disposed within the working sheath assembly and adapted lo permit a working instrument entry into the body cavity.

23. The endoscope of claim 22, wherein the working sheath segment al the distal end of the working sheath is actively flexible.

24. The endoscope of claim 22, including a working channel extension, associated with the face tip assembly which includes at least one protrusion lo guide the working instrument and to prevent impact of foreign matter upon the at least one optical image collector.

25. The endoscope of claim 24, wherein the at least two optical image collectors lie in a first plane which is disposed substantially perpendicular to the longitudinal axis of the working sheath assembly; and the al least one protrusion is disposed at the distal end of the working sheath forward of the first plane in which the at least two optical image collectors lie, whereby the at least two optical image collectors may view an operating tool passing forwardly beyond the at least one protrusion.

26. The endoscope of claim 25, wherein the plurality of input/output ports include at least one operating tool port, and the operating tool port lies in a second plane which is disposed substantially parallel with the first plane in which the at least one optical collector lies.

27. The endoscope of claim 26, wherein the first plane and the second plane are substantially coplanar.

28. The endoscope of claim 26, wherein the second plane is disposed in a spaced relationship from the first plane, toward the distal end of the working sheath.

29. The endoscope of claim 25, wherein the al least one protrusion has two peaks which extend forwardly toward a distal end of the face tip assembly.

30. The endoscope of claim 29, wherein the plurality of input/output ports include at least one operating tool port, having two sides, and the two peaks are spaced apart from each other, with a peak disposed adjacent each side of the operating tool port.

31. The endoscope of claim 30, wherein the peaks are disposed offset from the longitudinal axis, with a substantial portion of each peak disposed below the longitudinal axis.

32. An endoscope for viewing a portion of a body cavity, comprising:
a face tip assembly, having a plurality of input/output ports, associated with a working sheath assembly, the working sheath assembly being associated with a gripping handle and viewing assembly;
the working sheath assembly including a longitudinal axis, a working sheath having a distal end, and a working sheath segment disposed at the distal end of the working sheath for insertion into the body cavity;
at least two optical image collectors which produce a three dimensional image from within the body cavity;
at least one optical conductor, associated with the at least two optical image collectors, and adapted to transmit the three dimensional image to the gripping handle and viewing assembly;
at least one luminous conductor adapted to provide illumination to the body cavity; at least one working channel disposed within the working sheath assembly and
adapted to permit a working instrument entry Into the body cavity; and
a working channel extension, associated with the face tip assembly which includes at least one protrusion to guide the working instrument and to prevent impact of foreign matter upon the at least one optical image collector.

33. The endoscope of claim 32, wherein the at least one optical image collector lies in a first plane which is disposed substantially perpendicular to the longitudinal axis of the working sheath assembly; and the at least one protrusion is disposed at the distal end of the working sheath forward of the first plane in which the at least one optical image collector lies, whereby the at least one optical image collector may view an operating tool passing forwardly beyond the al least one protrusion.

34. The endoscope of claim 33, wherein the plurality of input/output ports include at least one operating tool port, and the operating tool port lies in a second plane which is disposed substantially parallel with the first plane in which the al least one optical collector lies.

35. The endoscope of claim 34, wherein the first plane and the second plane are substantially coplanar.

36. The endoscope of claim 34, wherein the second plane is disposed in a spaced relationship from the first plane, toward the distal end of the working sheath.

37. The endoscope of claim 32, wherein the face tip assembly includes at least two optical image collectors, each optical image collector being disposed in a first plane which is disposed substantially perpendicular to the longitudinal axis of the working sheath assembly.

38. The endoscope of claim 33, wherein the al least one protrusion has two peaks which extend forwardly toward a distal end of the face tip assembly.

39. The endoscope of claim 38, wherein the plurality of input/output ports include at least one operating tool port, having two sides, and the two peaks are spaced apart from each other, with a peak disposed adjacent each side of the operating tool port.

40. The endoscope of claim 39, wherein the peaks are disposed offset from the longitudinal axis, with a substantial portion of each peak disposed below the longitudinal axis.

41. The endoscope of claim 32, wherein the working sheath segment disposed al the distal end of the working sheath is actively flexible.

42. The endoscope of claim 41, wherein the actively flexible working sheath segment is disposed adjacent a passively flexible working sheath segment.

43. A method for viewing a portion of a body cavity, comprising the steps of:
providing an endoscopic type instrument having a face tip assembly associated with a working sheath assembly having a distal end and a proximal end, the working sheath assembly having an actively flexible working sheath segment at its distal end, and the proximal end of the working sheath assembly is associated with a gripping handle and viewing assembly;
providing an illumination source;
inserting the distal end of the working sheath assembly into a body cavity; and
manipulating the actively flexible working sheath segment to a desired angular deflection in order to properly view an area of interest in the body cavity.

44. The method of claim 43, including the step of: advancing a working tool through the endoscopic instrument, into the cavity, while substantially, simultaneously viewing its exit through the face tip assembly.

45. The method of claim 43, including the steps of:
Irrigating the area of interest; and
suctioning particulate matter from the body cavity.

46. The method of claim 43, including the step of viewing the relative location of the face tip assembly during insertion of the distal end of the working sheath assembly to properly position the working sheath assembly with respect to the area of interest.

47. An endoscope for viewing a portion of a body cavity, comprising:
a face tip assembly, having a plurality of input/output ports, associated with a working sheath assembly, the working sheath assembly being associated with a gripping handle and viewing assembly;
the working sheath assembly including a longitudinal axis, a working sheath having a distal end, and a working sheath segment disposed at the distal end of the working sheath for insertion into the body cavity;
one optical image collector to gather an image from within the body cavity;
one optical conductor, associated with the optical image collector, and adapted lo transmit the image to the gripping handle and viewing assembly;
at least one luminous conductor adapted to provide illumination to the body cavity;
at least one working channel disposed within the working sheath assembly and adapted to permit a working instrument entry into the body cavity; and
a working channel extension, associated with the face tip assembly which includes al least one protrusion to guide the working instrument and lo prevent impact of foreign matter upon the optical image collector.

48. The endoscope of claim 47, wherein the optical image collector lies in a first plane which is disposed substantially perpendicular lo the longitudinal axis of the working sheath assembly; and the at least one protrusion is disposed at the distal end of the working sheath forward of the first plane in which the optical image collector lies, whereby the optical image collector may view an operating tool passing forwardly beyond the at least one protrusion.

49. The endoscope of claim 48, wherein the plurality of input/output ports include al least one operating tool port, and the operating tool port lies in a second plane which is disposed substantially parallel with the first plane in which the optical collector lies.

50. The endoscope of claim 49, wherein the first plane and the second plane are substantially coplanar.

51. The endoscope of claim 49, wherein the second plane is disposed in a spaced relationship from the first plane, toward the distal end of the working sheath.

52. The endoscope of claim 47, wherein the optical image collector is disposed in a first plane which is disposed substantially perpendicular to the longitudinal axis of the working sheath assembly.

53. The endoscope of claim 48, wherein the at least one protrusion has two peaks which extend forwardly toward a distal end of the face tip assembly.

54. The endoscope of claim 53, wherein the plurality of input/output ports include al least one operating tool port, having two sides, and the two peaks are spaced apart from each other, with a peak disposed adjacent each side of the operating tool port.

55. The endoscope of claim 54, wherein the peaks are disposed offset from the longitudinal axis, with a substantial portion of each peak disposed below the longitudinal axis.

56. The endoscope of claim 47, wherein the working sheath segment disposed at the distal end of the working sheath is actively flexible.

57. The endoscope of claim 58, wherein the actively flexible working sheath segment is disposed adjacent a passively flexible working sheath segment.

58. An endoscope system with an ergonomic and intuitive gripping handle to observe a visually obscured portion of a body cavity comprising in a preferred embodiment:
a. an intuitive and ergonomic gripping handle having a described working sheath of different elastic properties.
b. an intuitive and ergonomic gripping handle with a preferred embodiment with a rotor or manipulator at its middle third to produce a coherent deflection at the active flexible section in such a manner that by turning or rotating it to the right, the active flexible segment deflects, turns or is directed to the right and vice versa for the left side.
c. An intuitive and ergonomic gripping handle that at its proximal end and below the longitudinal axis L of the endoscope is fed by a luminous conductor that can be used in parallel with a cable of the imaging apparatus of the connecting camera to the endoscope eyepiece and prevent encountered vectorial forces at the gripping handle when rotation of the endoscope becomes necessary.
d. A gripping gripping handle that at its middle third and only at the left side is provided with a brake for the distal active flexible end of the working sheath with different elastic properties.
e. The described brake at the gripping handle that do not fixate or freeze the active flexible segment of the working sheath with different elastic properties, but instead be moved as a gear mechanism step by step.
f. The intuitive and ergonomic gripping handle that at its distal third and below the longitudinal axis L is provided with an optional and interchangeable stabilizer of any type the design requires according to a particular use, required time of usage of the endoscope, and any other circumstances that could modify the design and requirements.

59. An endoscope system with an ergonomic gripping handle to observe a visually obscured portion of a body cavity comprising in a preferred embodiment:
a. an intuitive and ergonomic gripping handle having a described working sheath of different elastic properties.
b. an intuitive and ergonomic gripping handle with a preferred embodiment with a rotor or manipulator at its middle third to produce a coherent deflection at the active flexible section in such a manner that by turning or rotating it to the right, the active flexible segment deflects, turns or is directed to the right and vice versa for the left side.
c. An intuitive and ergonomic gripping gripping handle that at its proximal end and below the longitudinal axis L of the endoscope is fed by a luminous conductor that can be used in parallel with a cable of the imaging apparatus of the connecting camera to the endoscope eyepiece and prevent encountered vectorial forces at the gripping handle when rotation of the endoscope becomes necessary.
d. A gripping gripping handle that at its middle third and only at the left side is provided with a brake for the distal active flexible end of the working sheath with different elastic properties.
e. The described brake at the gripping handle that do not fixate or freezes the active flexible segment of the working sheath with different elastic properties, but instead be moved as a gear mechanism step by step.
f. The intuitive and ergonomic gripping handle that at its distal third and below the longitudinal axis L is provided with an optional and interchangeable stabilizer of any type the design requires according to a particular use, required time of usage of the endoscope, and any other circumstances that could modify the design and requirements.
g. The gripping handle at its proximal end is provided with two optical image collectors and/or two optical conductors in order to render a three dimensional image.

60. The endoscope of claim 1 that under the same concept can be utilized for other different medical areas, such as urology, general surgery, laparoscopic surgery, neurosurgery, orthopedics, plastic surgery and others that according to present evolution or in the future, requires an endoscope type concept such as the present invention.

61. The endoscope of claim 1 and 2 and under the same concept can be utilized altogether with other technologies and new developments such as "Natural Orifice Endoscopic Surgery" (NOTES). In such a manner as with the at present new technology like robotic surgery through adaptations with this same concept of the present invention and place it on earth planet or earth's surface as well as in the space, any space station, or the surface of any other planet or planetary satellite.

62. The endoscope of claim 1,2 and 3 that under the same concept can be used in Veterinary medicine.

63. The endoscope of claim 1,2,3 and 4 that can be used in the industry, space programs as well as any other machinery type or required situation.
